(19) **Europäisches Patentamt**
**European Patent Office**
**Office européen des brevets**

(11) **EP 3 377 162 B1**

(12) **EUROPÄISCHE PATENTSCHRIFT**

(45) Veröffentlichungstag und Bekanntmachung des Hinweises auf die Patenterteilung:
**11.08.2021  Patentblatt 2021/32**

(51) Int Cl.:
*A61M 16/04* *(2006.01)*     *A61M 25/10* *(2013.01)*

(21) Anmeldenummer: **16815926.7**

(22) Anmeldetag: **18.11.2016**

(86) Internationale Anmeldenummer:
**PCT/IB2016/001643**

(87) Internationale Veröffentlichungsnummer:
**WO 2017/085540 (26.05.2017 Gazette 2017/21)**

(54) **VORRICHTUNG ZUR DYNAMISCH DICHTENDEN OKKLUSION ODER RAUMFÜLLENDEN TAMPONADE EINES HOHLORGANS**

DEVICE FOR A DYNAMICALLY SEALING OCCLUSION OR A SPACE-FILLING TAMPONADE OF A HOLLOW ORGAN

DISPOSITIF POUR L'OCCLUSION ÉTANCHE DYNAMIQUE OU LE TAMPONNEMENT PAR COMBLEMENT D'UN ORGANE CREUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.11.2015   DE 102015014824**
**04.12.2015   PCT/IB2015/002309**

(43) Veröffentlichungstag der Anmeldung:
**26.09.2018   Patentblatt 2018/39**

(73) Patentinhaber: **Creative Balloons GmbH**
**68753 Waghäusel (DE)**

(72) Erfinder: **GÖBEL, Fred**
**69259 Wilhelmsfeld (DE)**

(74) Vertreter: **Küchler, Stefan**
**Stefan T. Küchler**
**Patentanwalt**
**Färberstraße 20**
**90402 Nürnberg (DE)**

(56) Entgegenhaltungen:
**EP-A1- 1 442 765        WO-A1-2005/120618**
**US-A- 3 794 043         US-A- 3 848 605**
**US-A- 4 501 273         US-A- 4 762 129**
**US-A- 5 029 591         US-A- 5 947 927**
**US-A1- 2007 277 830     US-A1- 2013 146 062**

**Beschreibung**

Die Erfindung richtet sich vorrangig auf eine Vorrichtung zum dichtenden Verschluss und/oder zur raumfüllenden Tamponade eines Hohlorgans oder einer sonstigen Kavität im menschlichen Körper, mit einem vollständig und residual ausgeformten Ballon, welcher der Wandung des abzudichtenden oder zu tamponierenden Organs mit einem möglichst konstant wirkenden Dichtungsdruck des Ballons anliegt, und mit einem isobar wirkenden Regler für den Fülldruck innerhalb des Binnenraums des Ballons, umfassend ein außerhalb des Körpers extrakorporal angeordnetes Volumenreservoir sowie eine Zuleitung zur kommunizierenden Verbindung des extrakorporalen Volumenreservoirs des Reglers mit dem Binnenraum des Ballons. Die erfindungsgemäße Vorrichtung dient bevorzugt zur Tamponade eines eigenmotilen oder korrespondierend motilen bzw. mobilen Organs und/oder zur dynamisch dichtenden Intubation eines Hohlorgans, insbesondere zur trachealen Intubation und Beatmung eines Patienten zur rasch volumenkompensierenden Dichtung der Trachea.

Ein grundsätzliches Problem beim dichtenden Verschluss bzw. der raumfüllenden Tamponade von Hohlorganen oder sonstigen Kavitäten im menschlichen Körper ergibt sich aus der spezifischen physiologischen Dynamik des jeweiligen Organs oder Raumes. Muskulös-bindegewebig begrenzte Organe weisen in der Regel eine weitgehend organeigene Motilität auf, können aber auch korrespondierenden Einwirkungen angrenzender motiler Strukturen ausgesetzt sein. Derartig eigenmotile oder korrespondierend motile Organe oder Räume erfordern zur effizienten Dichtung und/oder Tamponade einen besonderen, der jeweiligen Dynamik folgenden, funktionelle Schwankungen kompensierenden Regelmechanismus, der möglichst zeitsynchron Veränderungen des Organdurchmessers oder der Organform, des Organtonus bzw. des jeweilig herrschenden Organbinnendrucks möglichst fortwährend und bei möglichst geringem apparativen Aufwand ausgleicht.

Die Herausforderung einer dynamischen, sich zeitsynchron adaptierenden Ballondichtung oder -tamponade kann gut am Beispiel der menschlichen Luftröhre (Trachea) verbildlicht werden. Die Trachea ist eine aus knorpeligen, bindegewebigen und muskulösen Anteilen aufgebaute, rohrartige Struktur. Sie reicht vom unteren Abschnitt des Kehlkopfes bis zur Aufzweigung in die Hauptbronchien. Die vorderen und die seitlichen Portionen der Luftröhre sind dabei durch spangenartige, in etwa hufeisenförmige knorpelige Strukturen stabilisiert, die wiederum durch bindegwebige Lagen miteinander verbunden sind. Auf der dorsalen, offenen Seite der Knorpelspangen wird das Luftröhrenlumen durch die sogenannte Pars membranacea abgeschlossen, die aus durchgängig muskulös-bindegewebigem Material, ohne versteifende Elemente besteht. Ihr liegt dorsal wiederum die muskulös-bindegewebige Speiseröhre an, die ebenfalls keine knorpeligen Elemente enthält.

Das obere Drittel der Trachea befindet sich in der Regel außerhalb der Brusthöhle (Thorax), während die beiden unteren Drittel innerhalb der von Brustkorb und Zwerchfell abgegrenzten Brusthöhle liegen. Der thorakale Anteil der Trachea ist so in besonderer Weise den dynamischen Druckschwankungen in der Brusthöhle ausgesetzt, die sich im Rahmen der physiologischen Atemarbeit eines spontan atmenden oder auch assistiert spontan atmenden Patienten im Thorax einstellen. Entsprechendes gilt für die Speiseröhre (Ösophagus). Sie durchzieht den Thorax in ganzer Länge. Messungen des sogenannten intra-thorakalen Drucks, einem für die Beatmungsplanung wesentlichen Parameter, erfolgen standardmäßig durch druckaufnehmende Messvorrichtungen, die im Ösophagus platziert werden, da sich hier sich die atemmechanisch bedingten thorakalen Druckschwankungen, aufgrund des Weichteilcharakters des Organs, mit bestmöglicher Näherung erfassen lassen.

Bei der Einatmung (Inspiration) wird das thorakale Volumen durch die Hebung der Rippen und die simultane Senkung des Zwerchfells vergrößert, wodurch der im Thorax herrschende intra-thorakale Druck synchron abfällt. Dieser atemmechanisch in der Brusthöhle hergestellte Druckabfall führt in den thorakalen Anteilen der Trachea zu einer geringgradigen Weitung des trachealen Lumens. Bei der trachealen Intubation mit herkömmlichen Beatmungskathetern wird der tracheal dichtende Cuff typischerweise im Bereich des Übergangs des mittleren zum unteren Drittel der Luftröhre platziert. So positionierte Cuffs sind den beschriebenen, atemsynchron zyklischen Weitungen der Trachea exponiert, was korrespondierend zyklische Schwankungen des Cuff-Dichtungsdrucks zur Folge hat. Die wesentliche Aufgabe von Cuff-Ballonen besteht zum einen darin, die tiefen Luftwege gegen einströmende Sekrete des Rachens zu schützen sowie zum anderen in der Dichtung der unteren Luftwege bei einer Beatmung mit positiven Beatmungsdrucken. Die durch die thorakale Eigenatmung des Patienten verursachten Schwankungen des thorakalen Drucks können den Dichtungsdruck im Cuff in kritisch niedrige Bereiche bewegen, in denen eine suffiziente Dichtung gegen Sekrete des Rachens oder der Speisewege nicht mehr gewährleistet ist und der Patient beispielsweise Mageninhalt aspiriert.

Während die erreichbare Dichtungsleistung herkömmlicher Cuff-Ballons auf PVC-Basis sehr eng mit dem aktuell im Ballon herrschenden Fülldruck korreliert, zeigen besonders dünnwandig hergestellte Ballonkomponenten aus Polyurethan (PUR) im Verlauf des Druckzyklus eine deutlich bessere bzw. stabilere Dichtungseffizienz.

Besonders kritisch ist die Sekretdichtung trachealer Beatmungskatheter im Cuff-Fülldruckbereich von 5 bis 15 mbar. Dichtungsoptimierte Kathetertypen mit mikrodünnwandigen PUR-Cuffs gewährleisten zwar auch hier noch gute Dichtungsleistungen, die erreichbaren Resultate sind jedoch für einen infektionspräventiven Aspirationsschutz unzureichend.

Im Stand der Technik sind fülltfruckregulierende Vorrichtungen für tracheale Beatmungskatheter beschrieben, die in verschiedener technischer Ausführung versuchen, eine zeitsynchrone, dynamische Anpassung des Dichtungsdrucks im trachealen Cuff herzustellen. Die verschiedenen Technologien gehen dabei von der kombinierten Anwendung mit herkömmlich aufgebauten, konventionellen trachealen Beatmungskathetern aus. Die Befüllung der tracheal dichtenden Cuffs erfolgt bei diesen Bauweisen durch eine in den Schaft des Katheters extrudierte bzw. sonstig integrierte, sehr kleinlumige Befüllzuleitung, die in der Regel keinen ausreichend großen Volumenstrom des den Ballon mit Druck beaufschlagenden Mediums erlaubt, um eine Anpassung des tracheal dichtenden Fülldrucks mit ausreichender kleiner Latenz zu ermöglichen. Eine außerhalb des Körpers platzierte Vorrichtung zur prompten Volumenkompensation im tracheal dichtenden Cuff kann daher, nahezu unabhängig von der Art und Funktionsweise des Mechanismus, nur einen verzögerten, nur partiell dichtungsunwirksamen Volumenausgleich erreichen. Selbst technologisch aufwendige, für eine schnelle Regulation ausgelegte Systeme, wie beispielsweise das Gerät CDR 2000 der Firma Logomed GmbH, sind durch die im Verbund mit herkömmlichen Beatmungskathetern resultierende Trägheit hinsichtlich der erreichbaren Dichtungsleistung unzureichend.

Die EP 1 442 765 A1 offenbart einen bis in die Bronchien einführbaren Endotrachealtubus mit einem manschettenförmigen Ballon zur Abdichtung des Tubus gegenüber den Bronchien sowie mit einer Einrichtung, um die Manschette durch Einspeisen eines Fluids aufzublasen, wobei die Einrichtung einen extrakorporalen Pumpball aufweist, der über einen Schlauch mit dem Ballon kommuniziert. Der Pumpball weist eine flexible Hülle auf und kann manuell zusammengepresst werdem, während er sich beim Loslassen selbst expandiert. An seinem dem Ballon abgewandten Ende befindet sich ein Rückschlagventil, an dem Verbindungsschlauch zwischen dem Pumpball und dem Ballon ist dagegen nur ein manuell betätigbares Absperrventil vorgesehen. Zwischen diesem Absperrventil und dem Pumpballon wiederum ist ein Rohr mit einem verengten Innenquerschnitt eingefügt, wodurch die Strömungsqueschwindigkeit zwischen dem Pumpballon und dem Ballon grundsätzlich begrenzt wird. Da alle diese Elemente in Reihe geschalten sind, gibt es keine Bypassfunktion, und das Füllen des Ballons ist nur durch manuelles Betätigen des Pumpballons und selbst dann nur mit einer begrenzten Strömungsgeschwindigkeit möglich. Mit einer solchen Einrichtung ist es nicht möglich, die Fluidmenge in dem Ballon dynamischen Druckschwankungen innerhalb eines Patienten zeitsynchron nachzuführen.

Die in der WO 2005 / 120 618 A1 beschriebene Einrichtung dient dem Aufblasen eines Ballons, welcher im Bereich des distalen Endes eines in die Trachea eines Patienten einführbaren Beatmungstubus diesen manschettenförmig umgebend angeordnet ist. Hier ist anstelle eines Pumpballs ein Gummibalg vorgesehen, welcher an einem Anschluss eines Dreiwegeventils angeschlossen ist. Das Dreiwegeventil verfügt über drei Differenzdruckkammern, welche gemeinsam das Verhalten einer elstischen Membranscheibe des Ventils steuern. Im Normalzustand bei befülltem Ballon befindet sich das Dreiwegeventil jedoch in einem geschlossenen Gleichgewichtszustand, und erst bei Überschreiten vorgegebener Schaltschwellen durch bestimmte Differenzdrucke kann das Ventil in der einen oder anderen Richtung geöffnet werden. Durch diese Konstruktion, wobei das Ventil in einem Gleichgewichtszustand jegliche Strömungen zu oder von dem Ballon abriegelt, wird im Bedarfsfalle wertvolle Zeit vergeudet. Denn eine Strömung zu oder von dem Ballon wird erst nach extrakorporaler Sensierung eines eine Schaltschwelle überschreitenden Differenzdrucks und eine daraus folgende Umsteuerung des Ventils ermöglicht. Daher muss jeder Druckanstieg in einem Patienten zunächst zu einem vorübergehenden Überdruck des Ballons gegenüber dem an jenem anliegenden Bereich der Trachea führen, damit jener von dem Dreiwegeveritil erkannt wird und Anlass zur Öffnung eines dortigen Entlastungsventils gibt.

Der US 2013 / 0 146 062 A1 ist ein pädriatischer Trachealtubus mit einem manschettenförmigen Dichtungsballon offenbart. Ferner erkennt man eine mit dem Ballon zu dessen Befüllung verbundene, nach extrakorporal führende Füllleitung, Ein Regler, um den Ballonfülldruck ständig möglichst konstant zu halten, also auch im Falle von Druckschwankungen innerhalb des Patienten, ist diesem Dokument nicht zu entnehmen.

Die US 4,762,129 A offenbart einen Dilatationskatheter mit einem manschettenförmigen Ballon, jedoch keinen Regler zur Nachführung eines Druckes in dem Ballon.

Das die Erfindung initiierende Problem resultiert daraus, dass eine dichtungseffiziente, der Motilität eines abzudichtenden oder zu tamponierenden Hohlorgans eines Patienten zeitsynchron, also möglichst unverzögert folgende, Druckschwankungen kompensierende Dichtungstechnik im Stand der Technik bislang nicht zu finden ist.

Bei einer gattungsgemäßen Vorrichtung mit einem isobar wirkenden Regler für den Fülldruck innerhalb des Binnenraums des Ballons, umfassend ein außerhalb des Körpers extrakorporal angeordnetes Volumenreservoir sowie eine Zuleitung zur kommunizierenden Verbindung des extrakorporalen Volumenreservoirs des Reglers mit dem Binnenraum des Ballons, wird dieses Problem dadurch gelöst, dass die verbindende Zuleitung zwischen dem Ballon und dem Regler ein flußrichtendes Rückschlag-Ventil aufweist, welches den Rückschlag vom Ballon zu dem Volumenreservoir des Reglers verhindert, sowie ein parallel zu dem flussrichtenden Rückschlagventil angeordnetes, nichtflußrichtendes Drosselelement, welches den langsamen Volumenausgleich zwischen Ballon und Volumenreservoir ermöglicht.

Durch diese Struktur kann im Fall einer Vergrößerung des Volumens innerhalb des Hohlorgans rasch der Fülldruck innerhalb des dichtenden Ballons erhöht werden, weil dann das flußrichtende Rückschlagventil infolge des Druckabfalls in dem Ballon öffnet, während ansonsten, insbesondere bei einer Reduzierung des Volumens innerhalb des Hohlorgans, der Fülldruck innerhalb des Ballons nur allmählich abgebaut wird.

Die vorliegende Erfindung beschreibt eine neuartige Kathetertechnik, die eine besondere Bauweise der Zuleitung des Füllmediums zum tracheal dichtenden Cuff-Ballon aufweist, welche erstmals einen effizienten, ausreichend raschen Volumenstrom zwischen einem körper-externen Regelmechanismus und dem tracheal platzierten Cuff ermöglicht. Auf der Basis einer derartigen fluss-optimierten Zuleitung bzw. Kommunikation zwischen Regler und Cuff können technologisch einfache Vorrichtungen zur Volumen- bzw. Cuffdruckregulation zur Anwendung kommen, die ohne aufwendige Steuerungselektronik oder -mechanik eine atemsynchrone Dichtung des Cuffs erreichen und somit auch fest mit dem jeweiligen Katheter verbundene single-use Lösungen ermöglichen.

Die Erfindung beschreibt derartige single-use Komponenten zur isobaren Volumenkompensation, geht aber alternativ auch auf mögliche elektronische Lösungen der Cuffdruck-Stabilisierung im Verbund mit der erfindungsgemäßen Flussoptimierung des dichtenden Mediums ein.

Neben der optimierten Kommunikation des dichtenden Mediums zwischen der extrakorporal regelnden und der tracheal dichtenden Einheit beschreibt die Erfindung auch abgeleitete Bauweisen von Beatmungskathetern zur Gewährleistung möglichst großer Innenlumina für die möglichst widerstandsarme tracheale Beatmung bzw. Spontanatmung von Patienten.

Ferner beschreibt die Erfindung Bauweisen, die das Pooling von Sekret im Zwischenraum von Cuff und Stimmlippen vermeiden um zum anderen potentiell traumatisierende Relativbewegungen zwischen dem Katheterschaft und den Strukturen des Kehlkopfes vermeiden.

Neben fluss-optimierten, katheterschaft-integrierten Zuleitungen zwischen Regler und Cuff, beschreibt die Erfindung besondere tracheale Ballonkomponenten, die am distalen Ende des Beatmungskatheters fest und dicht schließend auf dem Schaftkörper aufgebracht sind und sich im Bereich des Übergangs vom thorakalen in den extra-thorakalen Abschnitt der Luftröhre, im Bereich der Stimmlippen, oder auch im Bereich des unteren. Rachens, annähernd auf das Außenmaß des Katheterschaftes verschlanken, aber zwischen der Hülle des so verschlankten proximalen Anteils des Ballonelementes und dem von der Hülle umschlossenen Schaft einen vorzugsweise allseitig freien Spalt lässt. Dieser spaltartige, um den Katheterschaft herum angelegte Zwischenraum ermöglicht einen besonders großlumigen, raschen Volumenfluss des dichtenden Mediums.

Am proximalen Ende des so verschlankten Ballonendes geht der Spaltraum in eine Schaftstruktur über, welche in entsprechender Weise für eine möglichst verzögerungsfreie, fluss-optimierte Zuleitung von Füllmedium ausgestattet ist bzw. über entsprechend fluss-optimierte, in den Schaftkörper integrierte zuleitende Kanäle verfügt.

Der Übergang des proximalen Ballonendes auf die aufnehmende Schaftstruktur erfolgt bevorzugt unterhalb der Stimmlippen (sub-glottisch), optional aber auch oberhalb der Stimmlippen (supra-glottisch).

Die konzentrische Anordnung des Katheterschaftes innerhalb eines verjüngten schlauchartigen Ballonsegmentes ist insbesondere im Bereich der Stimmlippen von Vorteil. Die schlauchartige Ballonhülle liegt hier den empfindlichen Strukturen des inneren Kehlkopfes schützend an und vermeidet eine direkte, mechanisch traumatisierende Wirkung des Katheterschaftes.

Als weitere Gestaltungsvariante einer tracheal dichtenden Ballonkomponente schlägt die Erfindung eine Cuff-in-Cuff Anordnung vor, wie diese bereits in EP 1061984 B1 vorgestellt wurde. Diese besondere Ausführung zweier ineinander geschachtelter Cuffs ermöglicht neben der trachealen Dichtung durch einen erfindungsgemäß stabilisierten inneren Ballon bei relativ höherem Dichtungsdruck, auch eine Tamponade des sub-glottischen Raumes durch einen äußeren Ballon bei einem relativ niedrigeren Tamponadedruck. Durch die extra-thorakale Positionierung der äußeren sub-glottisch oder auch glottisch tamponierenden Hülle im oberen Drittel der Luftröhre, übertragen sich die thorakalen Druckschwankungen nur moderat auf den herrschenden Tamponadedruck, wodurch dieser ohne relevanten Druckverlust in einem gewebeschonenden, sehr niedrigen Fülldruckbereich gehalten werden kann. Neben der geschachtelten Anordnung ist auch eine zwischenraumfreie, sequentielle Anordnung von tracheal dichtendem Cuff (distal) und sub-glottischem Tamponadeballon (proximal) denkbar.

Die Erfindung beschreibt ferner eine besondere für diese konzentrische oder sequentielle Anordnung ausgelegte Reservoireinheit mit einer Doppelballonanordnung zur separierten Einstellung zweier verschiedener Fülldrucke.

Der erfindungsgemäße Volumenfluss zwischen tracheal dichtendem Ballonkörper und extrakorporalem Regler kann annähernd effizient auch bei Trachealtuben und Tracheostomiekanülen üblicher Bauart mit endständigem Cuff-Ballon erreicht werden, bei denen der zuführende Kanal zum Cuff in die Schaftwandung des Katheterschaftes integriert ist und der zuleitende Kanal eine Querschnittfläche aufweist, die der einer kreisrunden Querschnittsfläche bei einem Kreisdurchmesser von mindestens 2 mm entspricht. Suffiziente Flüsse werden bei Querschnittsflächen mit einem Kreisdurchmesser von 3 bis 4 mm. Die erfindungsgemäße Wirkung setzt dabei entsprechend ausgelegte Durchmesser der zuführenden Schlauchleitung voraus.

Das erfindungsgemäße Prinzip der Stabilisierung eines Dichtungsdrucks durch flussoptimierte Zuleitung eines Füllmediums kann ebenfalls in vorteilhafter Weise bei den dichtenden Manschetten supra-glottischer Luftwege, sogenannten Larynxmasken, angewendet werden. Hier kann auch bei herkömmlich aufgebauten Dichtungskörpern, die die Maske über dem Kehlkopf fixieren und dichten, durch eine Kopplung des dichtendenden Manschettenelementes an eine regelnde Reservoireinheit und die Einbindung einer integrierten Ventil-Drossel Kombination eine entscheidende Verbesserung der Dichtungseffizienz und Anpassungsdynamik an die sich ändernden Toni im Bereich des Hypopharynx erreicht werden.

Neben Anwendungen im Bereich der Dichtung der oberen und unteren Luftwege kann das erfindungsgemäße Prinzip ferner bei trans-ösophagealen Sonden verwendet werden, die mit einem ösophageal dichtenden Ballonelement ausgestattet sind. Die Effizienz der Dichtung ist hier ebenfalls in hohem Umfang von der Fähigkeit zur möglichst raschen Volumenverschiebung zwischen dem extrakorporalen Reservoir und dem Dichtungsballon abhängig.

Weitere Merkmale, Einzelheiten, Vorteile und Wirkungen auf der Basis der Erfindung ergeben sich aus der folgenden Beschreibung bevorzugter Ausführungsbeispiele der Erfindung sowie anhand der Zeichnung. Hierbei zeigt:

Abb. 1    einen erfindungsgemäßen Trachealtubus mit durchgängig schaftintegrierter Zuleitung, im Verbund mit einer externen, volumen- bzw. druckregulierenden sowie einer flussrichtenden Vorrichtung;

Abb. 1a    ein beispielhaftes, Abb. 1 entsprechendes Schaftprofil im Querschnitt;

Abb. 2    einen Trachealtubus mit spaltraum-bildenden, verlängerten, proximalen Ballonende, das im sub-glottischen Bereich in den Schaftkörper übergeht;

Abb. 2a    eine entsprechende Ausführung zu Abb. 2, wobei das verlängerte proximale Ballonende jedoch im supra-glottischen Bereich mit dem Schaftkörper verbunden wird;

Abb. 2b    einen atraumatischen Übergang zwischen Schaftkörper und proximalem Ballonende;

Abb. 2c    vorteilhafte Größenverhältnisse des Spaltraums zu den Durchmessern des Tubenschaftes;

Abb. 2d    eine nutartige Präformation des Schaftkörpers zur Volumenverschiebung;

Abb. 2e    einen Querschnitt durch die in 2d gezeigte Präformation;

Abb. 3    einen einkammerigen Ballon, der sowohl tracheal dichtet als auch subglottisch tamponiert;

Abb. 3a    eine zweikammerige Anordnung mit einem tracheal dichtenden und einem sub- bis supra-glottisch tamponierenden Ballon;

Abb. 4    einen Trachealtubus mit einem zweiteiligen Schaftkörper, dessen supraglottischer Abschnitt besonders stabil als Beissschutz ausgeführt ist und dessen sich nach distal anschließender Schaft ein einlumiges, innendurchmessermaximiertes Segment aufweist;

Abb. 4a    ein triangulär ausgeformtes Schaftsegment zur Positionierung in der Glottis;

Abb. 5    eine Trachealkanüle mit nach extrakorporal verlängertem proximalen Ballon;

Abb. 6    einen kombinierten Ventil-/Drosselmechanismus;

Abb. 7    eine beispielhafte Regler- bzw. Reservoirkomponente;

Abb. 7a    eine zur in Abb.7 beschriebene Komponente zugehörige Druck-Volumenkurve;

Abb. 7b    einen für zweikammerige Systeme ausgelegten Reservoir- bzw.-Reglerballon;

Abb. 8    einen erfindungsgemäßen, zuleitungsoptimierten Trachealtubus mit einem Sensorelement im Bereich des tracheal dichtenden Ballonsegmentes und einer mit dem Sensor in einem Regelkreis angeordneten Reglereinheit;

Abb. 9    eine Larynxmaske mit angeschlossener Reglereinheit; sowie

Abb. 10    eine trans-ösophageale Sonde mit angeschlossener Reglereinheit.

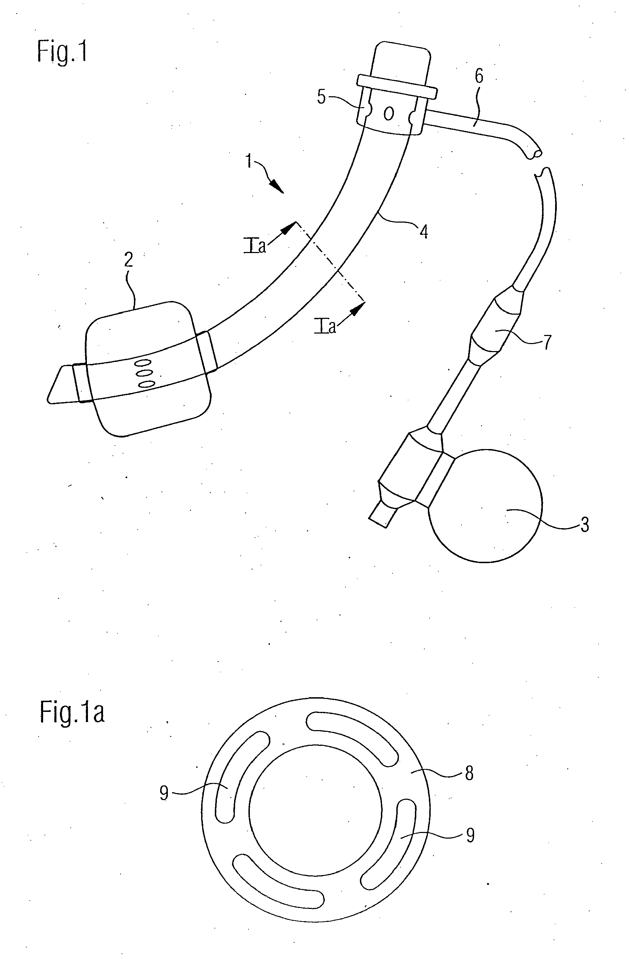

Abb. 1 beschreibt in einer beispielhaften Gesamtübersicht verschiedener funktionsoptimierender Komponenten einen trachealen Beatmungskatheter 1 (Trachealtubus) zur dynamischen trachealen Dichtung bei zyklisch alternierenden thorakalen Drucken durch flussoptimierte Verschiebung eines mit Druck beaufschlagten Füllmediums, zwischen einem tracheal positionierten Dichtungsballon 2 (Cuff) und einem extrakorporalen Regler- bzw. Reservoirelement 3.

Formgebung und Dimension der Vorrichtung der erfindungsgemäßen Vorrichtung entsprechen dabei weitgehend einem herkömmlichen Trachealtubus. Der tracheal dichtende Ballon ist am distalen Ende des Tubus mit seinen beiden Enden dicht schließend mit dem ballon-tragenden Katheterschaft verbunden. Der Schaftkorpus 4 weist bevorzugt eine in den Schaft integrierte, großlumige bzw. mehrlumige Zuleitung zum Cuff auf. Am proximalen Schaftende 5 werden die jeweiligen zuleitenden Lumina zusammengeführt und von dort aus mit einer groß-lumigen Zuleitung 6 an das Reglerelement 3 angebunden. Zur Vermeidung von schnellen retrograden Entleerungen zum Reservoir hin, sowie für den verzögerten Druck- bzw. Volumenausgleich zwischen den endständigen Kompartimenten ist in die Zuleitung eine kombinierte Ventil-Drossel Funktion 7 integriert. Im Verbund der kommunizierenden Volumina von Ballon, Zuleitung, Ventil-Drossel und Reservoir- bzw. Reglerelement entsteht ein gemeinsamer Binnenraum, in dem ein konstanter, durch das Reservoir bzw. den Regler definierter Druck aufrechterhalten wird. Als bevorzugtes Medium zur Befüllung des kommunizierenden Binnenraumes kommt im Rahmen der Erfindung Luft zur Anwendung.

Die im Rahmen der Erfindung beschriebene Technologie zur möglichst widerstandsminimierten, optimal raschen Volumenverschiebung, bei gleichzeitig möglichst geringen Druckgradienten zwischen einem tracheal positionierten Ballon und einer extrakorporalen Reglereinheit, soll eine druckstabilisierende Volumenkompensationen innerhalb des tracheal dichtenden Ballons ermöglichen, die im optimalen Falle nach nicht mehr als 10 bis 20 Millisekunden nach Einsetzen eines atemmechanisch ausgelösten Druckabfalls abgeschlossen, sind.

Abb. 1a zeigt einen beispielhaftes, mehrlumig ausgeführtes Schaftprofil 8 im Querschnitt. Die in die Schaftwandung integrierten Zuleitungs-Lumina 9 sind im Sinne einer möglichst geringen Vergrößerung des Schaftaußendurchmessers bevorzugt flach bzw. mit einer möglichst geringen radialen Höhe ausgeführt. Als Schaftmaterial kommt bei der in Abb. 1 und 1a gezeigten Basis-Bauweise bevorzugt PVC des Durometerbereiches Shore 80A bis 90A in Frage.

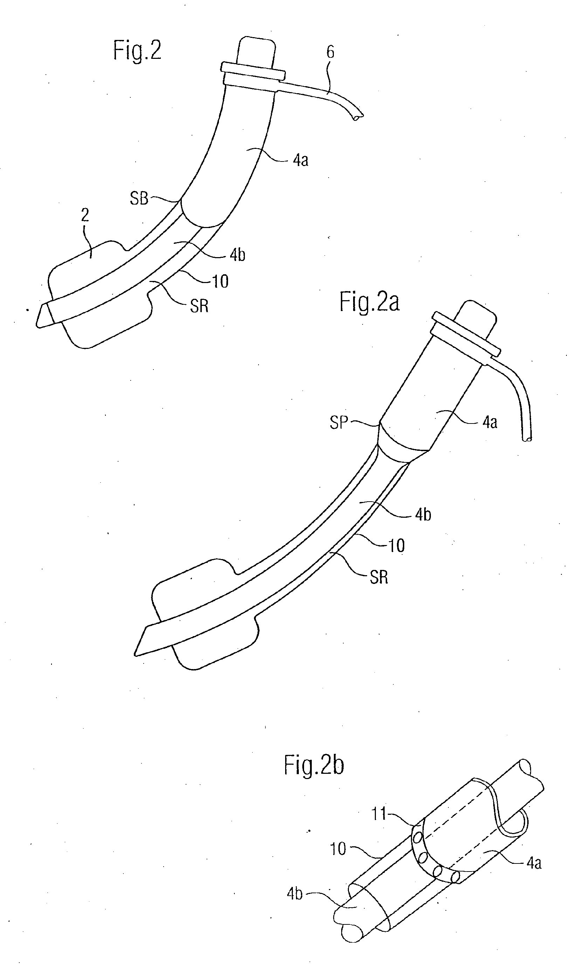

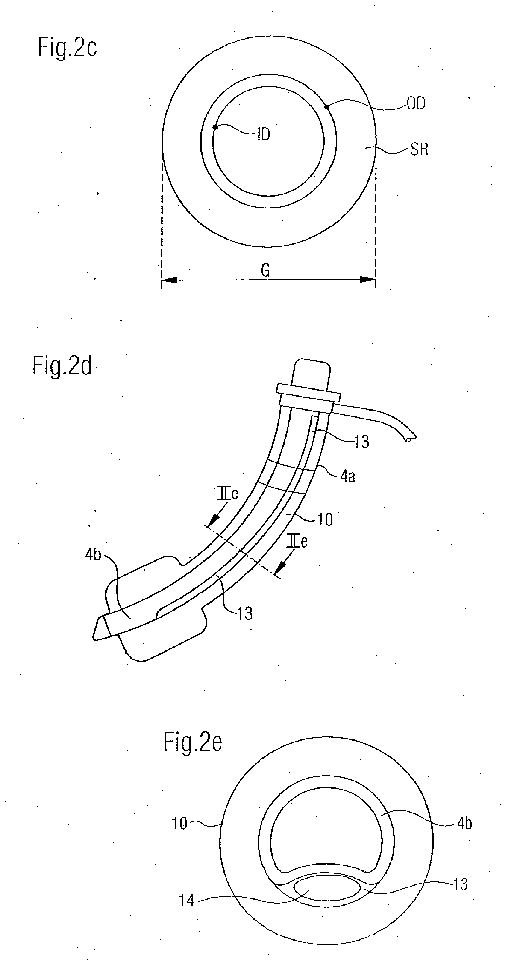

Abb. 2 zeigt einen erfindungsgemäßen Trachealtubus, der über einen am distalen Tubenende befestigten, tracheal dichtenden Ballon 2 verfügt, dessen verjüngtes, proximales Ende 10 bis in den sub-glottischen Bereich SB weitergeführt und dort vom proximalen Schaftanteil 4a aufgenommen bzw. mit diesem dicht schließend verbunden wird. Während der distale Schaftanteil 4b einlumig ausgeführt ist, weist der proximale Anteil bevorzugt ein mehrlumiges Profil entsprechend Abb. 1a auf. Wie in Abb. 1 erfolgt die Zuleitung des Füllmediums zum proximalen Ballonende so in flussoptimierter Weise. Das schlauchartig verjüngte Ballonende 10 schafft einen großlumigen Spaltraum SR zwischen der Schlauchhülle und dem Schaft, der frei mit dem dichtenden Ballonkorpus kommuniziert und so sehr vorteilhafte, erfindungsgemäß niedrige Flusswiderstände ermöglicht.

Abb. 2a zeigt einen Abb. 2 entsprechenden Trachealtubus, wobei der Übergang bzw. die Verbindung des nach proximal verlängerten, einen Spaltraum zum Schaft ausbildenden Ballonendes 10, im supra-glottischen Bereich SP, also oberhalb der Stimmlippen erfolgt. Der flusswirksame Querschnitt des sich bei dieser Bauform ergebende Spaltraum SR soll mindestens dem flusswirksamen Querschnitt der Summe der schaftintegrierten zuleitenden Lumina entsprechen, wobei dieser wiederum mindestens dem flusswirksamen Querschnitt der Schlauchverbindung zwischen dem Tubenschaft und der Reglereinheit entsprechen soll.

Das nach proximal verlängerte, schlauchartige Ballonende 10 kann bei der Formung des tracheal dichtenden Ballonkorpus direkt aus diesem hervorgehen oder auch als separat hergestelltes schlauchartiges Element an das proximale Ende des dichtenden Korpus, zum Beispiel durch Verklebung, angefügt werden. Es besteht ebenfalls bevorzugt aus Polyurethan niedriger Wandstärke von 10 bis 50 μm und besonders bevorzugt 10 bis 30 μm.

Abb. 2b zeigt den sub- oder supra-glottischen Übergang vom proximalen Ballonende 10 zum proximalen Schaftende 4a in einer bespielhaften Ausführung. Der Übergang 11 des proximalen 4a zum distalen 4b Schaftende ist dabei bevorzugt atraumatisch konisch ausgeführt. Die schaftintegrierten Lumina 9 münden über die konische Anschrägung in den Spaltraum SR.

Abb. 2c schlägt für die Dimensionierung des Spaltraumes im Bereich der proximalen Ballonverlängerung 10 besondere dimensionale Verhältnisse der volumenverschiebenden Querschnittfläche des Spaltraumes SR zur Querschnittfläche des Beatmungslumens ID sowie zur Gesamtquerschnittsfläche OD des Katheters vor. Die in Abb. 2 und 2a dargestellte Spaltfläche S, resultiert aus der Differenz der Durchmesser der durch die Hüllenwandung im Bereich 10 vorgegebenen Querschnittfläche G und der durch die Schaftaußenfläche begrenzten Querschnittfläche OD des Schaftkörpers. Die Spaltfläche S soll dabei 1/10 bis 5/10 der Querschnittsfläche G, besonders bevorzugt 2/10 bis 3/10 der Querschnittsfläche G betragen. Bezogen auf die Querschnittsfläche ID des Innenlumens des Schaftkörpers soll die Spaltfläche S 2/10 bis 6/10 der Querschnittsfläche ID, besonders bevorzugt 3/10 bis 4/10 der Querschnittsfläche ID betragen.

Abb. 2d zeigt ein besonderes bauliches Detail des Schaftkörpers 4, das insbesondere bei der Ausführung der Vorrichtung nach Abb. 2a einen unbehinderten Volumenfluss über die anatomisch enge Stimmlippenebene hinweg gewährleistet. Die dargestellte Vertiefung 13 kann beispielsweise aus einer oder mehreren, in der Schaftlängsachse nutartig verlaufenden Einziehungen bestehen. Die Vertiefung soll gewährleisten, dass auch im Falle eines passageren oder dauerhaften Verschlusses des volumenverschiebenden Spaltraumes ein ausreichend rascher Volumenstrom zwischen Regler und dichtendem Ballon kommunizieren kann. In die Vertiefung kann beispielsweise ein netzartiges Rohrgeflecht 14 oder ein entsprechendes Schlauchelement mit siebartiger Perforation eingelegt sein, welche die Vertiefung offenhält. Die Vertiefung kann bei bevorzugter Positionierung über den Scheitel der großen Kurvatur zur Stabilisierung des Tubenschaftes gegen Knickung und Verwindung beitragen.

Abb. 2e zeigt die Vertiefung im Schaftkörper 4 im Querschnitt.

Für eine beispielhafte quantitative Berechnung der Strömungsverhältnisse in einem in den Abbildungen 2 und 2a definierten, zylindermantelförmigen Spaltraum SR, insbesondere auf die Druckverhältnisse im distalen, tracheal dichtenden Ballonabschnitt 2, sollen folgende Platzhaltergrößen verwendet werden:

| | |
|---|---|
| $V_1$ | Volumen des tracheal dichtenden Ballons 2 |
| $P_1$ | Druck im tracheal dichtenden Ballon 2 |
| $\rho_1$ | Fülldichte im tracheal dichtenden Ballon 2 |
| $M_1$ | Luftmasse im tracheal dichtenden Ballon 2 |
| $V_2$ | Volumen des extrakorporalen Reservoirs 3 |
| $P_2$ | Druck im extrakorporalen Reservoir 3 |
| $\rho_2$ | Fülldichte im extrakorporalen Reservoir 3 |
| $m_2$ | Luftmasse im extrakorporalen Reservoir 3 |

Für die Luftmassen $m_1$, $m_2$ gilt:

$$m_1(t) = m_{1,0} + \int_{\tau=0}^{\tau=t} S_{m,1}(\tau)\, d\tau \qquad (1)$$

$$m_2(t) = m_{2,0} - \int_{\tau=0}^{\tau=t} S_{m,2}(\tau)\, d\tau \qquad (2)$$

Dabei meint $S_{m,v}$ die Luftströmung zu dem betreffenden Ballon 2, 3 als Luftmassenströmung.

Gemäß dem Gesetz von Hagen-Poiseuille gilt für die massenbezogene Fluidströmung durch eine Leitung mit dem Innenradius R und einer Länge l:

$$S_{m,1} = \frac{\rho_1 \cdot \pi\, (p_2 - p_1) \cdot R^4}{8\, \eta \cdot l} \qquad (3)$$

$$S_{m,2} = \frac{\rho_2 \cdot \pi \, (p_1 - p_2) \cdot R^4}{8 \, \eta \cdot l} \qquad\qquad (4)$$

Dabei ist $\eta$ die dynamische Viskosität des strömendes Gases. Für Luft gilt:
$\eta$ beträgt bei 273 K 17,1 $\mu$Pa $\cdot$ s
Ferner gilt aufgrund der thermischen Zustandsgleichung idealer Gase im Ballon 2:

$$\eta_1 = \rho_1 \cdot R_S \cdot T_1 \qquad\qquad (5)$$

und im Reservoir 3:

$$\eta_2 = \rho_2 \cdot R_S \cdot T_2 \qquad\qquad (6)$$

Dabei ist $R_s$ die individuelle oder spezifische Gaskonstante, welche für Luft den Wert 287,058 J/(kg$\cdot$K) hat.
$T_V$ ist die Temperatur in dem Ballon 2 bzw. in dem Reservoir 3.
Für eine Temperatur von 23 °C bzw. 296 K beträgt der Faktor

$$k = R_{S,Luft} \cdot T_{23°C} = 85 \cdot 10^3 \ J/(kg \cdot K) \qquad\qquad (7)$$

Im Folgenden soll angenommen werden, dass die Temperatur sowohl in dem Ballon 2 als auch im Reservoir 3 konstant 23° C beträgt:

$$T_1 = T_2 = 296 \ K.$$

Dann gilt:

$$p_1 = \rho_1 \cdot k \qquad\qquad (8)$$

$$p_2 = \rho_2 \cdot k \qquad\qquad (9)$$

Damit ergibt sich durch Einsetzen von Gleichung (3) in Gleichung (1):

$$m_1(t) = m_{1,0} + \int_{\tau=0}^{\tau=t} \frac{p_1 \cdot \pi \cdot R^4}{8\,\eta \cdot l}(p_2 - p_1)\,d\tau \qquad (10)$$

Mit der Gleichung (8) folgt daraus:

$$m_1(t) = m_{1,0} + \int_{\tau=0}^{\tau=t} \frac{p_1 \cdot \pi \cdot R^4}{8\,\eta \cdot l \cdot k}\,p_1\,(p_2 - p_1)\,d\tau \qquad (11)$$

Ferner gilt im Ballon 2 :

$$\frac{m_1}{V_1} = \rho_1 = \frac{p_1}{k} \qquad (12)$$

Daher lässt sich in Gleichung (11) für die Masse $m_1$ schreiben:

$$m_1 = \frac{V_1 \cdot p_1}{k} \qquad (13)$$

Daraus folgt:

$$\frac{V_1}{k} \cdot p_1(t) = \frac{V_1}{k} \cdot p_{1,0} - \int_{\tau=0}^{\tau=t} \frac{\pi \cdot R^4}{8\,\eta \cdot l \cdot k} \cdot [p_1^2 - p_1 p_2]\,d\tau \qquad (14)$$

Die gesamte Gleichung lässt sich mit $V_1/k$ kürzen. Eine Differenzierung auf beiden Seiten liefert:

$$\frac{dp_1}{dt} = -\frac{\pi \cdot R^4}{V_1 \, 8 \, \eta \, l} \cdot [p_1^2 - p_1 p_2] \qquad (15)$$

Es handelt sich hierbei um eine Bernoullische Differenzialgleichung von der Form:

$$x' = -a \cdot x \cdot (x - b), \qquad (16)$$

wobei

$$a = \frac{\pi \cdot R^4}{V_1 \, 8 \, \eta \, l} \qquad (17)$$

$$b = p_2 \qquad (18)$$

Im Folgenden soll angenommen werden, dass das Reservoir 3 erheblich größer ist als der Ballon 2:

$$V_2 >> V_1$$

Daraus folgt, dass der Druck $p_2$ im Reservoir 3 näherungsweise etwa konstant bleibt, auch wenn sich der Druck $p_1$ im Ballon 2 kurzzeitig ändert. Unter dieser Annahme sind die Koeffizienten a und b aus der Bernoullischen Differenzialgleichung (16) konstant, und die Lösung der Bernoullischen Differenzialgleichung lautet:

$$x(t) = \frac{b}{1 - e^{-abt - bc_1}} \qquad (19)$$

Die Integrationskonstante $c_1$ lässt sich wie folgt bestimmen:

$$p_1(t) = \frac{p_2}{1 - e^{-ap_2 t - p_2 c_1}} \qquad (20)$$

Für t = 0 muss gelten:

$$p_1(t) = p_{1,0} \qquad (21)$$

Daraus folgt:

$$p_{1,0} = \frac{p_2}{1 - e^{-p_2 c_1}} \qquad (22)$$

$$\frac{p_2}{p_{1,0}} = 1 - e^{-p_2 c_1} \qquad (23)$$

$$e^{-p_2 c_1} = \frac{p_{1,0} - p_2}{p_{1,0}} \qquad (24)$$

$$-p_2 c_1 = \ln \frac{p_{1,0} - p_2}{p_{1,0}} \qquad (25)$$

$$c_1 = -\frac{1}{p_2} \cdot \ln \frac{p_{1,0} - p_2}{p_{1,0}} = \frac{1}{p_2} \cdot \ln \frac{p_{1,0}}{p_{1,0} - p_2} \qquad (26)$$

Eingesetzt in Gleichung (2) liefert dies:

$$\frac{p_1(t)}{p_{1,0}} = \frac{p_2}{p_{1,0} - [p_{1,0} - p_2] \cdot e^{-(\pi R^4 p_2 t)/(8 V_1 \eta l)}} \qquad (27)$$

Diese Gleichung ist von der Form:

$$\frac{p_1(t)}{p_{1,0}} = \frac{p_2}{p_{1,0} - [p_{1,0} - p_2] \cdot e^{-t/\tau}} \qquad (28)$$

mit

$$\tau = (8V_1\eta l) / (\pi R^4 p_2) \qquad (29)$$

Für geringe Druckschwankungen im Ballon 2 gilt bspw.:

Ferner gilt für $t = \tau$: $p_{1,0} \approx 0{,}99\, p_2$

$$e^{-t/\tau} = e^{-1} \approx 0{,}37$$

Und für $t = 4\tau$:

$$e^{-t/\tau} = e^{-4} \approx 0{,}018$$

In Gleichung (28) eingesetzt liefert dies:

$$\frac{p_1(t=4\tau)}{0{,}99\, p_2} = \frac{p_2}{0{,}99\, p_2 - (0{,}99\, p_2 - p_2) \cdot 0{,}018}$$

bzw. :

$$p_1(t=4\tau) = p_2 \cdot \frac{0{,}99}{0{,}99 + 0{,}018 \cdot 0{,}01} = 0{,}9998 \cdot p_2$$

Dies ist nur noch 2 % von der anfänglichen Abweichung.

Bei der Anwendung im Rahmen der Atmung ist zu beachten, dass ein Atemzyklus etwa 3 sec. dauert. Damit währenddessen der Cuff nicht undicht wird, sollte diese Ausgleichszeit $t = 4\tau \approx 8$ ms sein.

Daraus folgt:

$$\tau = 2 \cdot 10^{-3} \text{ s} = \frac{8 \cdot 5 \cdot 10^{-6} \text{ m}^3 \cdot 0,2 \text{ m} \cdot 17,1 \cdot 10^{-6} \text{ Pas}}{\pi \cdot R^4 \cdot 10^5 \text{ Pa}}$$

Dabei wurde angenommen:

$V_1 = 5 \text{ cm}^3$
$l = 20 \text{ cm}$
$p_2 = 10^5 \text{ Pa}$

Damit ergibt sich:

$$R^4 = 0,7 \cdot 10^{-12} \text{ m}^4,$$

also:
$R = 0,91 \cdot 10^{-3} \text{ m} \approx 1 \text{ mm}.$

Da die Strömungsverhältnisse in einem zylindermantelförmigen Hohlraum deutlich schlechter sind als in einem zylindrischen Hohlraum, sollte der radiale Querschnitt eines zylindermantelförmigen Hohlraums erheblich größer sein. Außerdem wurde bei der obigen Berechnung die Zuleitungen 6 und 9 ebenfalls vernachlässigt, die jedoch auch einen nicht unerheblichen Strömungswiderstand darstellen. Deshalb sollte die radiale Höhe des zylindermantelförmigen Spaltraums 10 wenigstens 2 mm betragen oder besser noch 3 bis 4 mm.

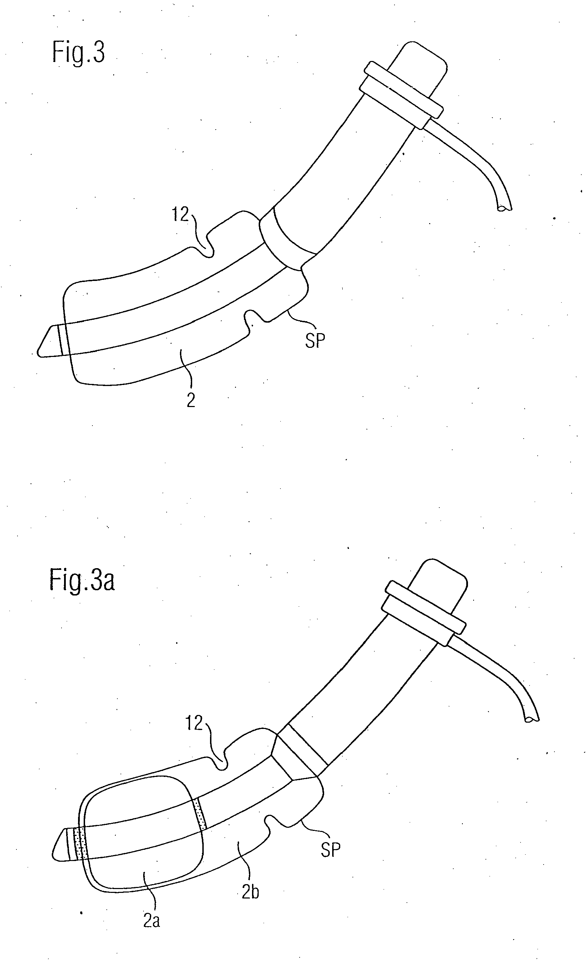

Abb. 3 zeigt einen Trachealtubus mit einem über die Stimmlippenebene hinaus verlängerten Ballonelement 2. Das Ballonelement soll, entsprechend den weiteren im Rahmen der Erfindung beschriebenen Ausführungsformen, vorzugsweise aus ausgeformtem Folienmaterial bestehen, welches im Durchmesser derart residual bemessen ist, dass es zur Dichtung des trachealen Lumens nicht gedehnt werden muss und sich der Schleimhaut des Organs unter Einfaltung der überschüssigen Ballonhülle weitgehend spannungslos anschmiegt. Die Erfindung bevorzugt polyurethan-basierte Ballonfolien, die im Bereich des tracheal dichtenden Ballonsegmentes eine Wandstärke von bevorzugt 5 bis 20 $\mu$m, weniger bevorzugt von 20 bis 50 $\mu$m aufweisen.

Für das tracheal dichtende Ballonelement kommen erfindungsgemäß bevorzugt Polyurethane der Härten Shore 70A bis 95A bzw. 55D bis 65D zur Verwendung. Besonders bevorzugt kommen Shore-Härten des Bereiches 85A bis 95A zum Einsatz.

Während das Ballonelement im einfachen Falle für die Dichtung im Bereich des Übergangs des unteren zu mittleren trachealen Drittel dimensioniert ist, wie dies bei konventionellen Trachealtuben oder Trachealkanülen üblich ist, kann das tracheal dichtende Ballonsegment im Rahmen der Erfindung auch nach proximal verlängert werden und über die Stimmlippen hinaus in den Bereich des supra-glottischen, unteren Rachens reichen. Der Korpus des Ballonelementes 2 wird dabei bevorzugt zylindrisch ausgeformt. Er kann im Bereich der Stimmlippenebene mit einer zirkulären Verjüngung 12 zur Aufnahme der Stimmlippen versehen sein.

Die nach proximal verlängerte Ausführung des tracheal dichtenden Ballons gestattet ein besonders großes Ballonvolumen, welches in der Lage ist, eine gewisse druckerhaltende Pufferwirkung zu entwickeln, wenn es im trachealen Abschnitt des Ballonkorpus zu atemmechanisch bedingten Vergrößerungen des trachealen Querschnitts bzw. zu einer nachlassenden transmuralen Kraftwirkung auf den tracheal dichtenden Ballon kommt. Reicht das proximale Ballonende aus dem Thorax heraus, ist dieses extra-thorakale Segment der thorakalen Atemmechanik nicht ausgesetzt, was den dämpfenden Effekt der extra-korporalen Volumenreserve entsprechend unterstützt, und die erfindungsgemäße, dynamisch wirkende, dichtungserhaltende Funktion der Vorrichtung weiter verbessert.

Ferner kann durch die große Kontaktfläche eines nach proximal verlängerten, tracheal dichtenden Ballons ein größtmöglicher Migrationsweg für Sekrete bzw. darin enthaltene Erreger ermöglicht werden.

In der Abb. 3a wird ein Abb. 3 entsprechender Tubus vorgestellt, der zwei ineinander geschachtelte Cuffs beinhaltet und neben der trachealen Dichtung durch einen inneren Ballon 2a bei relativ höherem Dichtungsdruck, auch eine Tamponade des sub-glottischen Raumes durch einen äußeren Ballon 2b bei einem relativ niedrigeren Tamponadedruck ermöglicht. Neben der konzentrischen Anordnung ist auch eine, sequentielle, zwischenraumfreie Anordnung von tracheal dichtendem Cuff (distal) und sub-glottischem Tamponadeballon (proximal) denkbar.

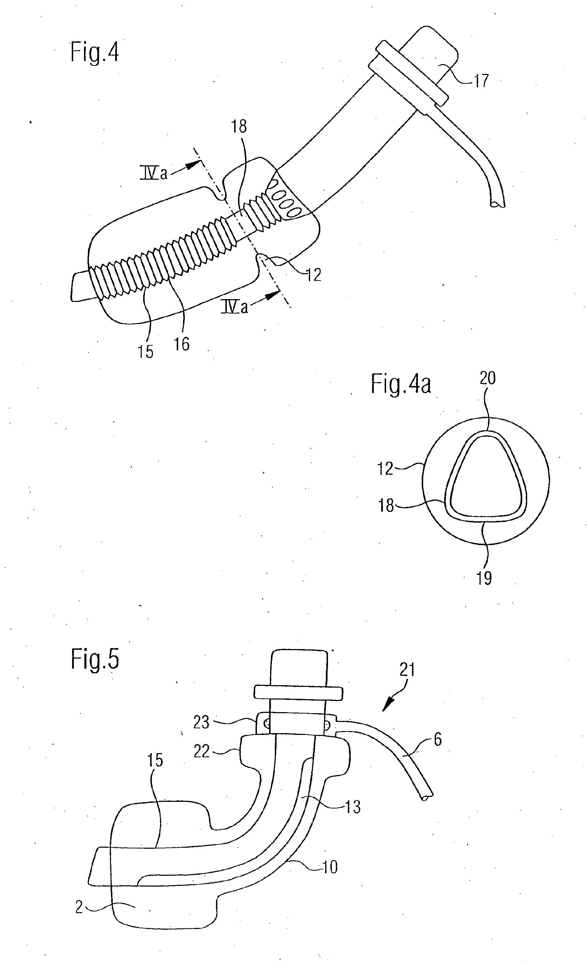

In der in Abb. 4 dargestellten Ausführung besteht der für die tracheale Beatmungsvorrichtung verwendete Katheterschaft 4 ebenfalls aus Polyurethan (PUR), da PUR bei entsprechender Kombination von Materialhärte und Bearbeitung, wie z.B. einer Profilierung der Schaftoberfläche die Option zur Herstellung sehr dünnwandiger Schäfte mit dennoch guter Knickstabilität aufweist. Im Vergleich zu Materialalternativen wie Silikon oder PVC, werden so größere, den Flusswiderstand des Atemgases verbessernde Innenlumina erlaubt. Da der Schaft in der Schaftwandung keine Füll-Leitung zum dichtenden Cuff integriert, wie dies bei konventionellen Tubenschäften üblich ist, kann das ventilationswirksame Innenlumen des Schaftes entsprechend vergrößert und damit der Flusswiederstand bei der Atmung oder Beatmung des Patienten optimal weit reduziert werden.

Der besonders dünnwandige, einlumig ausgeführte Schaftkörper 15 wird zu Stabilisierung des Schaftlumens und zur Ermöglichung weitgehend spannungsloser axialer Biegungen des Schaftes bei der Platzierung in den Luftwegen mit einer Welligen Korrugation 16 seiner Oberfläche ausgestattet. Im bevorzugten Fall sollten axiale Biegungen des Schaftes von 90 bis 180 Grad ohne relevante Lumeneinengung und ohne relevante auf die Gewebe anlastende elastische, für Polyurethan typische, potentiell traumatische Rückstellkräfte möglich sein. Der Schaft kann so in optimaler Weise, Bewegungen des Patienten bzw. Relativbewegungen zwischen Schaft und Patienten folgen.

Bei Innendurchmessern des Schaftes von 7-10 mm kann bei der Kombination einer Wandungsstärke von ca. 0,3 bis 0,5 mm, einer Shore Härte von ca. 95A bis 75D, einem peak-to-peak Wellungsabstand von 0,3 bis 0,8 mm sowie einer Wellungsamplitude von 0,5 bis 1 mm ein entsprechend knickstabiler, lumenoptimierter Schaft hergestellt werden.

Die Korrugation kann sich auf den trachealen Abschnitt des Tubusschaftes beschränken, aber auch bis in den sub-glottischen Bereich oder über die gesamte Schaftlänge hinweg bis zum proximalen Ende des Schaftes und dem dortig angebrachten Konnektor 17 reichen.

Im Falle der korrugierten Ausführung des Schaftes 4 kann bei Verwendung einer wechselbaren Innenkanüle, wie diese bei Tracheostomiekanülen üblich sind, eine Innennkanüle mit kongruent korrugiertem Profil verwendet werden, deren Wellung sich in die Wellung der Außenkanüle optimal, einfügt und die Außenkanüle so entsprechend, bei geringer kombinierter Wandstärke von Außen- und Innenkanüle vorteilhaft stabilisierend versteift und beispielsweise so Teilwandstärken von 0,3 bis 0,5 mm der Außenkanüle und 0,1 bis 0,3 mm der Innenkanüle ermöglicht.

Die Abbildung zeigt eine kombinierte Ausführung des Schaftkörpers, wobei dessen tracheales und glottisches Segment aus einlagigem, innendurchmesser-optimierten Schaftmaterial besteht, und im supra-glottischen Segment in einen massiv ausgeführten, beispielsweise spritgegossenem PVC-Anteil übergeht, welches wie in Abb.1a dargestellt, mit mehrfachen Lumina 9 zur Zuleitung zum Ballonelement versehen sein kann. Insbesondere kann der sub-glottische Anteil durch einen eventuellen armierenden oder mehrschichtigen Aufbau derart verstärkt werden, dass er als Beißschutz dient und so, insbesondere bei vigilanten Patienten, den Lumenverschluß des Tubus bei Kieferschluß vermeidet.

Abb. 4a zeigt einen Querschnitt durch die Ausführungsform in Abb. 5 in ihrem glottischen Segment 18. Das Profil des Schaftes kann hier triangulär gestaltet sein, so dass es sich mit seiner Basis 19 auf die dorsale Fläche der Stimmlippenebene lebt, mit seinem Apex 20 nach ventral weist, und sich so optimal der triangulären Öffnung der Stimmlippen anpasst.

Abb. 5 zeigt eine beispielhafte Anwendung der Erfindung bei einer Tracheostomiekanüle 21. In analoger Weise zur Gestaltung bei Trachealtuben, wird das proximale Ballonende 10 hier durch das Stoma zur Luftröhre geleitet. Es kann dabei vor dem Stoma eine wulstartige Erweiterung 22 ausbilden. Die proximale Erweiterung kann als wulstartige Erweiterung ausgebildet sein, die einem Konnektor 23 aufsitzt, der über eine ringartig dichtende Lippe 23 die freie Verschieblichkeit des Konnektors auf dem Schaft erlaubt. Die proximale Erweiterung kann so zum Stoma hin verschoben werden, und die Vorrichtung so an die jeweilige Halsanatomie angepasst werden.

Zur Sicherstellung des ausreichend raschen Volumenstroms des Füllmediums zwischen allen intra-korporalen Anteilen des der Kanüle aufsitzenden Ballons 2 wird dieser vorzugsweise mit einer nutartigen, optional armierten Vertiefung versehen, wie diese in Abb. 2c beschrieben ist.

Im Sinne eines möglichst großen Innendurchmessers und damit möglichst geringen Ätmungs- bzw. Beatmungswiderstandes ist der Schaftkörper 4 der Kanüle hier ebenfalls bevorzugt aus dünnwandigem, wellig profiliertem PUR, wir in Abb. 4 beschrieben, hergestellt.

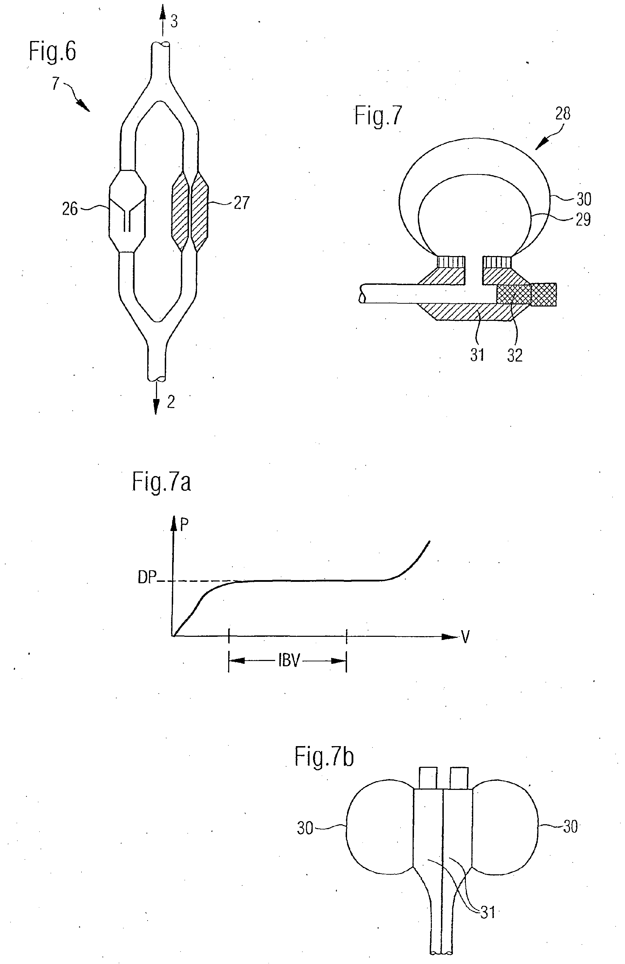

Abb. 6. Um plötzliche, retrograde, potentiell dichtungskritische Entleerungen des Ballonvolumens. zum Regle bzw. zum Reservoir hin zu vermeiden, wie diese beispielsweise beim mehrfachen Husten des Patienten in kurzer Sequenz erfolgen können, kann die verbindende Zuleitung 6 zwischen dem Schaft und der regelnden Reservoireinheit mit einem flussrichtenden Ventil 26 ausgestattet sein, welches den raschen Rückschlag von Füllmedium verhindert. Das Ventil soll dabei derart konstruiert sein, dass es den offenen, anterograden Volumenstrom vom Regler zum Ballon hin möglichst wenig beeinträchtigt.

Um durch das Ventil 26 verursachte Poolungs-Effekte von Medium im Ballon zu vermeiden, wird das Ventil bevorzugt mit einer nicht-flussgerichteten, nach beiden Seiten hin offenen Bypass-Drossel 27 ausgestattet, die einen langsamen, verzögerten Druck- bzw. Volumenausgleich zwischen den beiden endständigen Kompartimenten Ballon 2 und Regler 3 ermöglicht. Im einfachsten Ausführungsfalle wird die jeweilige, dichtende Ventilfläche mit einer kleinen Bohrung bzw. Öffnung versehen, die einen entsprechenden gedrosselten Volumenstrom erlaubt.

Abb. 7 beschreibt eine beispielhafte Ausführung einer kombinierten Regler- bzw. Reservoir-Einheit 3. Die Einheit weist einige konzeptionelle Merkmale eines sogenannten Lanz-Reglers auf (siehe US....). Die wesentliche funktionelle Komponente der Einheit 3 besteht wie bei der Einheit nach Lanz aus einem besonderen volumendehnbaren Ballonblase 28 aus hoch-elastischem Material, welches von einer gewissen vorgeformten, beispielsweise sphärischen Grund- bzw. Ruhefigur 29, durch Befüllung in einen gedehnte Arbeitsfigur 30 übergeht. Bei Verwendung geeigneter Materialien kann ein bestimmtes Expansionsverhalten der Ballonblase erreicht werden, wobei sich das Volumen bei isobarer Druckentwicklung im Ballon vergrößert. Die zugehörige, konzeptionell angestrebte Druck-Volumenkurve ist in Abb. 7a dargestellt. Die Ballonblase besteht vorzugsweise aus einem natürlichen, latex-ähnlichen Material oder aus einem synthetischen Stoff, wie Isopren-verwandten Material. Die Ballonblase sitzt einem Sockelgehäuse 31 auf, in das in bevorzugter Weise ein Einwegeventil 32 für die Befüllung mit Luft eingebaut ist.

Abb.7a zeigt eine zu Abb. 7 exemplarische Druck-Volumenkurve, die über einen bestimmten Volumenbereich VP hinweg einen konstanten Druck herstellt. Somit kann aus dem expandierten Ballon 30 heraus Volumen zum Ballon 2 abfließen, ohne dass sich durch den Volumenabfluss im Reservoir ein Druckverlust einstellt, das Druckplateau DP also verlassen wird. Für Anwendungen dieser Reglertechnologie bei Beatmungstuben soll der so einstellbare isobare Volumeneberich IBV in etwa dem frei ausgeformten Volumen des tracheal dichtenden Ballons entsprechen.

Abb.7b zeigt eine besondere doppelkammerige Reservoirballon-Anordnung DR, bei der zwei ballon-basierte Reservoirs nach Abb. 7 und 7a auf einem einzigen gemeinsamen Grundgehäuse 33 montiert werden. Die eine Kammer ermöglicht dabei einen trachealen Dichtungsdruck von 25 bis 30 mbar, während die andere die Einstellung eines Tamponadedrucks von 5 bis 15 mbar, beispielsweise für die sub-glottische Tamponade, wie in Abb. 3a beschrieben, ermöglicht.

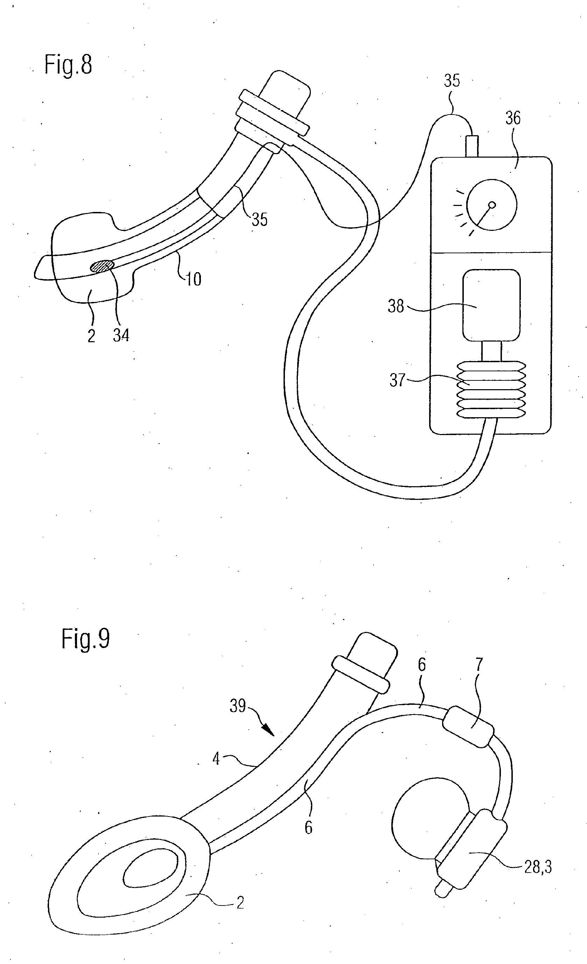

Abb. 8 zeigt einen Trachealtubus, der im Inneren des tracheal dichtenden Ballonsegmentes 2 mit einem druckaufnehmenden bzw. druckmessenden Fühlerelement 34 versehen ist. Der Druckfühler ist in bevorzugter Ausführungsweise ein elektronisches Bauelement, welches sein Messsignal über eine Kabelleitung 35 an einen elektronisch gesteuerten Regler 36 weiterleitet. Das Fühlerelement besteht vorzugsweise aus einem Absolutdrucksensor. Es können vorzugsweise Fühler auf der Basis von Dehnungsmessstreifen oder piezzo-elektrischer Sensoren verwendet werden. Der Regler 36 weist beispielsweise ein balgartiges oder kolbenartiges Reservoir 37 auf, welches durch einen Antrieb 38 betätigt, entweder Volumen zum Ballon 2 hin verschiebt oder aus dem Ballon 2 entnimmt, der Antrieb kann beispielsweise aus einem Schrittmotor bestehen oder als linear magnetischer Antrieb aufgebaut sein. Die Steuerung des Reglers ist derart ausgelegt, dass Abweichungen des Fülldrucks im Bereich des dichtenden Ballonsegmentes 2 sofort durch eine entsprechende Volumenverschiebung kompensiert werden bzw. der Fülldruck auf einem am Regler einstellbaren Sollwert SW konstant gehalten wird. Die Stabilisierung des dichtenden Ballondrucks erfolgt mit diesem Verfahren bereits zu einem Zeitpunkt, der optimal früh vor dem Einsetzen des maschinellen Beatmungshubes bzw. dem tatsächlichen Volumenfluss von Atemgas in die Lunge des Patienten liegt. Dies ist besonders bei Patienten relevant, die nach langer kontrollierter maschineller Beatmung erhöhte Atemarbeit aufwenden müssen, um eine nicht ausreichend volumendehnbare Lunge bis zu einem Punkt aufzuspannen, der einen effektiven Volumenstrom in die Lunge auslöst. In dieser Phase der isometrischen Anspannung der Lunge innerhalb des Thorax bzw. des mit der Anspannung der versteiften Lunge einhergehenden Druckabfalls innerhalb des Brustkorbs kann es zu aspirationsverursachenden Abfällen des Ballonfülldrucks kommen.

Im Gegensatz zu einem mechanischen Regler 3 einfachen Bautyps, welcher ein isobares Reservevolumen von vorzugsweise 20 bis 35 mbar zur Verfügung stellt, kann bei der beschriebenen elektronischen Regelung ein Druck aufgebaut werden, der den tracheal unkritischen Dichtungsdruck von 20 bis 35 mbar kurzzeitig überschreitet und so eventuellen vom Patienten ausgelösten Druckspitzen im trachealen Ballon dichtend entgegenwirken kann.

Abb. 9 zeigt eine sogenannte Larynxmaske 39, die an eine regelnde Reservoireinheit 3 mit flussoptimierter Zuleitung 6,9 sowie einer optionalen, erfindungsgemäßen Ventil-Drossel Kombination 7 ausgestattet ist. Das den Kehlkopf im Bereich des Hypopharynx dichtende, in der Regel toroide bzw. ringförmig geschlossene Ballonelement 2 ist dabei vorzugsweise aus einem mikro-dünnwandigen PUR-Material hergestellt, dass den hier vorgeschlagenen Materialien zur Herstellung des dichtenden Ballons trachealer Beatmungskatheter entspricht. Die Reglerkomponente ist bei der Anwendung im Hypopharynx derart ausgelegt, dass sich innerhalb des kommunizierenden Binnenraumes ein dichtendes Druckplateau DP von ca. 50 bis 60 mbar einstellt

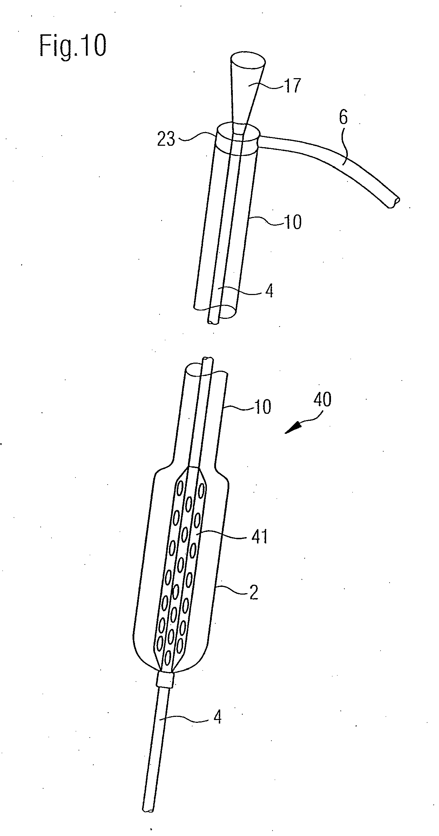

Abb. 10 zeigt eine trans-ösophageale Sonde 40 zur Zu- oder Ableitung von Substanzen oder Medien in den Magen oder duch den Magen hindurch, die mit einem ösophageal dichtenden Ballonelement 2 ausgestattet ist. Das proximale Ballonende 10 kann bis in den Bereich des extrakorporalen Konnektors 17 verlängert sein. Dort kann es in dicht schließender Weise mit einem auf dem Schaft frei verschiebbaren dichtenden Abschlusselement 23 verbunden sein, welches wiederum in eine lumen-große Zuleitung 6 übergeht, welche an einen erfindungsgemäßen Regelmechanismus 3 angeschlossen ist. Die Reglerkomponente stellt bei der ösophagealen Dichtung innerhalb des kommunizierenden Binnenraumes ein dichtendes Druckplateau DP von ca. 20 bis 30 mbar ein.

Zum verbesserten stationären Verbleiben des tamponierend dichtenden Ballonsegmentes im Ösophagus, kann dieses mit einem im osophagealen Bereich mit einem nicht kollabierbaren Profil 41 versehen werden, welches im Falle einer persiataltischen Kontraktion des Ösophagus Volumen aus den Ballonsegmenten vor der Kontraktionswelle, durch das oder unter dem Profil hindurch in Bereiche abgeleitet wird, die bereits von der Welle wieder frei gegeben sind. Somit kann ein Aufpilzen von Füllmedium vor der Kontraktionswelle verhindert werden, was den magenwärts gerichteten Transport der gesamten Vorrichtung zur Folge hätte. Entsprechende Profile sind bereits in der EP 0929339 B1 beschrieben.

**Patentansprüche**

1. Vorrichtung zum dichtenden Verschluss oder zur raumfüllenden Tamponade von Hohlorganen oder sonstigen Kavitäten im menschlichen Körper, mit einem Ballon (2), welcher vollständig und residual ausgeformt ist und der Wandung des abzudichtenden oder zu tamponierenden Organs mit einem möglichst konstant wirkenden Dichtungsdruck des Ballons (2) anliegt, und mit einem isobar wirkenden, Regler für den Fülldruck innerhalb des Binnenraums des Ballons (2), umfassend ein außerhalb des Körpers extrakorporal angeordnetes Volumenreservoir sowie eine Zuleitung (6) zur kommunizierenden Verbindung des extrakorporalen Reglers mit dem Binnenraum des Ballons (2), **dadurch gekennzeichnet, dass** die verbindende Zuleitung (6) zwischen dem Ballon (2) und dem Regler

   a) als zuleitender Kanal ausgebildet ist, der eine Querschnittsfläche aufweist, die einer kreisrunden Querschnittsfläche mit einem Kreisdurchmesser von mindestens 2 mm entspricht,
   b) ein flußrichtendes Rückschlag-Ventil (26) aufweist, welches den Rückschlag vom Ballon (2) zu dem Volumenreservoir verhindert,
   c) sowie ein parallel zu dem flußrichtenden Rückschlag-Ventil (26) angeordnetes, nicht-flußrichtendes Drosselelement (27), welches den langsamen Volumenausgleich zwischen Ballon (2) und Volumenreservöir ermöglicht.

2. Vorrichtung nach Anspruch 1, **dadurch gekennzeichnet, dass** die verbindende Zuleitung (6) zwischen dem Ballon (2) und dem Regler als zuleitender Kanal ausgebildet ist, der eine Querschnittsfläche aufweist, die einer kreisrunden Querschnittsfläche mit einem Kreisdurchmesser von 3 bis 4 mm entspricht.

3. Vorrichtung nach Anspruch 1 oder 2, umfassend einen in das Hohlorgan einführbaren Tubus mit einem primären Lumen als Zugang durch das oder zu dem betreffende(n) Hohlorgan, sowie einen diesen Tubus zwecks Abdichtung gegenüber dem Hohlorgan manschettenförmig umgebenden Ballon mit wenigstens einem sekundären Lumen zur Befüllung des Ballons.

4. Vorrichtung nach einem der Ansprüche 1 bis 3, **dadurch gekennzeichnet, dass** der Ballon

   a) einen radial erweiterten, distalen Bereich zur Abdichtung aufweist sowie einen sich daran anschließenden und demgegenüber radial verjüngten, proximalen Bereich als Umhüllung des (der) sekundären Lumens (Lumina) zur Befüllung des distalen Abdichtungs-Bereichs, und/oder
   b) mit unterschiedlichen Außendurchmessern in seinem distalen und seinem proximalen Bereich präformiert ist.

5. Vorrichtung nach einem der vorhergehenden Ansprüche, **dadurch gekennzeichnet, dass** in dem Ballon (2) ein radial verjüngter Bereich für die Stimmritze eingeformt ist.

6. Vorrichtung nach einem der vorhergehenden Ansprüche, **dadurch gekennzeichnet, dass** im proximalen Bereich des Ballons (2) ein einziges sekundäres Lumen vorgesehen ist, welches das primäre Lumen konzentrisch außen umgibt.

7. Vorrichtung nach einem der vorhergehenden Ansprüche, **dadurch gekennzeichnet, dass** der proximale Bereich des Ballons (2) sich nicht bis zu dem proximalen Ende des Tubus erstreckt, sondern vorher endet.

8. Vorrichtung nach einem der vorhergehenden Ansprüche, **dadurch gekennzeichnet, dass** der Ballon (2) oder der proximale Bereich des Ballons (2) an einer Stirnseite eines schlauchförmigen Elements endet, in welchem sich das primäre Lumen als lichte Öffnung radial innerhalb des Schlauchmantels fortsetzt, während sich das sekundäre Lumen in Form eines oder mehrerer in den Schlauchmantel selbst eingeformter Kanäle fortsetzt, wobei vorzugsweise der minimale Gesamtquerschnitt aller in den Schlauchmantel als sekundäre Lumina eingeformten Kanäle gleich oder größer ist als der maximale Querschnitt des ringförmigen sekundären Lumens im proximalen Bereich des Ballons.

9.  Vorrichtung nach einem der vorhergehenden Ansprüche, **dadurch gekennzeichnet, dass** sich im Bereich des proximalen Tubusendes ein ringförmiger Sammelkanal befindet, mit welchem alle sekundären Lumina oder alle in den Schlauchmantel als sekundäre Lumina eingeformten Kanäle kommunizieren.

10. Vorrichtung nach einem der vorhergehenden Ansprüche, **dadurch gekennzeichnet, dass** im Bereich des Tubusendes oder an dem ringförmigen Sammelkanal ein mit allen sekundären Lumina kommunizierender Anschluss für einen Befüllungsschlauch vorgesehen ist.

11. Vorrichtung nach einem, der vorhergehenden Ansprüche, **dadurch gekennzeichnet, dass** das extrakorporale Volumenreservoir (9)

    a) in frei entfaltetem Zustand ein größeres Volumen aufweist als der intrakorporale Ballon (4) oder als der manschettenförmige Dichtungsballon im distalen Bereich des Tubus, und/oder
    b) mit einem konstanten oder näherungsweise konstanten Druck beaufschlagt wird, beispielsweise durch ein Gewicht oder ein Federelement.

12. Vorrichtung nach einem der vorhergehenden Ansprüche, **gekennzeichnet durch** eine Einrichtung zur aktiven Steuerung oder Regelung des Drucks in dem extrakorporalen Volumenreservoir, welche derart ausgebildet sein kann, dass der Druck in dem intrakorporalen Ballon (2) oder in dem manschettenförmigen Dichtungsballon konstant gehalten wird.

13. Vorrichtung nach Anspruch 12, **gekennzeichnet durch** eine Messeinrichtung für den Druck in dem intrakorporalen Ballon (2) oder in dem manschettenförmigen Dichtungsballon zwecks Istwert-Vorgabe für einen Regelkreis, der auf den Druck in dem extrakorporalen Volumenreservoir einwirkt.

14. Vorrichtung nach Anspruch 12 oder 13, **dadurch gekennzeichnet, dass** das extrakorporale Volumenreservoir (9) als balgartiges oder kolbenartiges Reservoir (37) ausgebildet ist, welches durch einen Antrieb (38) betätigbar ist, entweder um Volumen zum Ballon (2) hin zu verschieben oder aus dem Ballon (2) zu entnehmen.

15. Vorrichtung nach einem der vorhergehenden Ansprüche, **dadurch gekennzeichnet, dass** der Ballon (2)

    a) ringförmig geschlossen oder toroid ausgebildet ist, und/oder
    b) als Larynx-Maske ausgebildet ist, insbesondere zur Auflage auf dem Hypopharynx.

16. Vorrichtung nach einem der vorhergehenden Ansprüche, **gekennzeichnet durch** ein nicht kollabierbares Profil (41) im Inneren des Ballons (2) oder Schaftes, welches im Falle einer peristaltischen Kontraktion des Hohlorgans einen Teil des Füllmediums aus den Ballonsegmenten distal zu der Kontraktionswelle in Bereiche proximal zu der Kontraktionswelle ableitet, wobei das nicht kollabierbare Profil mit dem Ballon (2) und/oder dessen Zuleitung und/oder Füll-Lumen kommuniziert.

17. Vorrichtung nach einem der vorhergehenden Ansprüche, **dadurch gekennzeichnet, dass** das flußrichtende Rückschlag-Ventil (26) oder das nicht-flußrichtende Drosselelement (27) oder beide extrakorporal angeordnet ist oder sind.

## Claims

1.  Device for the sealing occlusion or for the space-filling tamponade of hollow organs or other cavities in the human body, comprising a balloon (2), which is fully and residually formed and abuts the wall of the organ to be occluded or tamponaded under a sealing pressure of the balloon (2), which is as constant as possible, and comprising an isobarically acting regulator of the filling pressure within the interior of the balloon (2), the regulator comprising a volume reservoir arranged extracorporeally outside of the body as well as a supply line (6) for communicatively connecting the extracorporeal regulator o the interior of the balloon (2), **characterized in that** the connecting supply line (6) between the balloon (2) and the regulator

    a) is formed as a supply channel having a cross-sectional area which is equivalent to a circular cross-sectional area having a circle diameter of at least 2 mm,

b) comprises a flow-directing check valve (26) that prevents the reverse flow from the balloon (2) to the volume reservoir,

c) as well as a nonflow-directing throttle element (27) arranged in parallel to the flow-directing check valve (26), which allows a slow volume compensation between the balloon (2) and the volume reservoir.

2. Device according to claim 1, **characterized in that** the connecting supply line (6) between the balloon (2) and the regulator is formed as a supply channel having a cross-sectional area which is equivalent to a circular cross-sectional area having a circle diameter of 3 to 4 mm.

3. Device according to claim 1 or 2, comprising a tube which can be inseerted into a hollow organ, said tube comprising a primary lumen as an access through or to the relevant hollow organ, as well as a balloon surrounding said tube like a cuff for the purpose of sealing it against the hollow organ, and comprising at least one secondary lumen for the filling of the balloon.

4. Device according to one of claims 1 to 3, **characterized in that** the balloon

a) comprises a radially extended distal section for sealing purposes, and, adjacent thereto, a radially tapered proximal section, as a jacket for the secondary lumen (lumina) for the filling of the distal sealing section, and/or

b) is preformed with different outer diameters in its distal and its proximal section.

5. Device according to one of the preceding claims, **characterized in that** a radially tapered section for the glottis is preformed in the balloon (2).

6. Device according to one of the preceding claims, **characterized in that** a single secondary lumen concentricaly surrounding the primary lumen on its outside is provided in the proximal section of the balloon (2).

7. Device according to one of the preceding claims, **characterized in that** the proximal section of the balloon (2) does not extend up to the proximal end of the tube, but terminates in front of that.

8. Device according to one of the preceding claims, **characterized in that** the balloon (2) or the proximal section of the balloon (2) terminates at an end face of the tubular element, in which the primary lumen extends radially inside the tube jacket as a clear opening, whereas the secondary lumen extends in the form of one or multiple channels formed into the tube jacket, wherein the minimum total cross-section of all channels formed into the tube jacket as second lumina is equal to or greater than the maximum cross-section of the circular secondary lumen in the proximal section of the balloon.

9. Device according to one of the preceding claims, **characterized in that** an annular manifold is arranged in the area of the proximal end of the tube, wherein all secondary lumina or all channels formed into the tube jacket as secondary lumina communicate with the annular manifold.

10. Device according to one of the preceding claims, **characterized in that** a fitting for a filling tube communicating with all secondary lumina is provided in the area of the end of the tube or at the annular manifold.

11. Device according to one of the preceding claims, **characterized in that** the extracorporeal volume reservoir (9)

a) exhibits in a freely unfolded state a larger volume than the intracorporeal balloon (4) or a sealing balloon formed as a cuff in the distal area of the tube, and/or

b) experiences a constant or nearly constant pressure, for example through a weight or a spring element.

12. Device according to one of the preceding claims, **characterized by** an equipment for active control or regulation of the pressure in the extracorporeal volume reservoir, which can be formed in such a manner that the pressure in the intracorporeal balloon (2) or in the cuff-shaped sealing balloon is kept constant.

13. Device according to claim 12, **characterized by** a measuring system for the pressure in the intracorporeal balloon (2) or in the cuff-shaped sealing balloon, in order to specificate the actual value of a control loop, which acts upon the pressure in the extracorporeal volume reservoir.

**14.** Device according to claim 12 or 13, **characterized in that** the extracorporeal volume reservoir (9) is formed as a club-shaped or as a bellows-shaped reservoir (37), which is operable through a drive (38), in order to either move a volume to the balloon (2) or to remove it from the balloon (2).

**15.** Device according to one of the preceding claims, **characterized in that** the balloon (2)

> a) is formed annularly closed or of a toroidal shape, and/or
> b) is formed as a larynx mask, especially for a placing on the hypopharynx.

**16.** Device according to one of the preceding claims, **characterized by** a non-collapsible profile (41) in the interior of the balloon (2) or the shaft, which, in case of a peristaltic contraction of the hollow organ, drains a part of the filling medium from the balloon segments distally to the contraction wave into areas proximal to the contraction wave.

**17.** Device according to one of the preceding claims, **characterized in that** flow-directing check valve (26) or the nonflow-directing throttle element (27) or both is/are arranged extracorporeally.


**Revendications**

**1.** Dispositif pour la fermeture étanche ou le tamponnement par comblement d'organes creux ou autres cavités dans le corps humain avec un ballonnet (2) lequel est entièrement et résiduellement formé et qui repose contre la paroi de l'organe à étanchéifier ou à tamponner avec une pression d'étanchéification du ballonnet (2) ayant une action la plus constante possible, et contenant un régulateur à action isobare pour la pression de remplissage à l'intérieur de l'espace interne du ballonnet (2), comprenant un réservoir volumique extracorporel disposé à l'extérieur du corps ainsi qu'une conduite d'alimentation (6) pour la liaison communicante du régulateur extracorporel avec l'espace interne du ballonnet (2), **caractérisé en ce que** la conduite d'alimentation (6) de liaison entre le ballonnet (2) et le régulateur

> a) est réalisée sous forme de canal d'alimentation qui présente une surface de section qui correspond à une surface de section circulaire ayant un diamètre de cercle d'au moins 2 mm,
> b) comporte un clapet anti-retour à direction de flux (26) qui empêche le retour du ballonnet (2) au réservoir volumique,
> c) ainsi qu'un élément d'étranglement sans direction de flux (27), disposé parallèlement au clapet anti-retour à direction de flux (26) qui permet la compensation volumique lente entre le ballonnet (2) et le réservoir volumique.

**2.** Dispositif selon la revendication 1, **caractérisé en ce que** la conduite d'alimentation (6) de liaison entre le ballonnet (2) et le régulateur est réalisée sous forme de canal d'alimentation qui présente une surface de section qui correspond à une surface de section circulaire ayant un diamètre de cercle de 3 à 4 mm.

**3.** Dispositif selon la revendication 1 ou 2, comprenant un tube pouvant être introduit dans l'organe creux avec une lumière primaire en tant qu'accès par ou à l'organe creux concerné, ainsi qu'un ballonnet entourant sous forme de manchette ce tube à des fins d'étanchéification par rapport à l'organe creux avec au moins une lumière secondaire pour le remplissage du ballonnet.

**4.** Dispositif selon l'une des revendications 1 à 3, **caractérisé en ce que** le ballonnet

> a) présente un zone distale radialement élargie pour l'étanchéification ainsi qu'une zone proximale qui y est adjacente et rétrécie radialement par rapport à celle-ci en tant qu'enveloppe de la (des) lumière(s) secondaire(s) pour le remplissage de la zone d'étanchéification distale, et/ou
> b) est préfaçonné en ayant des diamètres extérieurs différents dans sa zone distale et sa zone proximale.

**5.** Dispositif selon l'une des revendications précédentes, **caractérisé en ce qu'**une zone radialement rétrécie est moulée dans le ballonnet (2) pour l'orifice glottique.

**6.** Dispositif selon l'une des revendications précédentes, **caractérisé en ce qu'**une seule lumière secondaire est prévue dans la zone proximale du ballonnet (2), lumière secondaire qui entoure concentriquement la lumière primaire à l'extérieur.

**7.** Dispositif selon l'une des revendications précédentes, **caractérisé en ce que** la zone proximale du ballonnet (2) ne s'étend pas jusqu'à l'extrémité proximale du tube, mais se termine avant.

**8.** Dispositif selon l'une des revendications précédentes, **caractérisé en ce que** le ballonnet (2) ou la zone proximale du ballonnet (2) se termine au niveau d'une face frontale d'un élément tubulaire dans lequel la lumière primaire se poursuit sous forme d'ouverture intérieure radialement à l'intérieur de la gaine de tuyau flexible, tandis que la lumière secondaire se poursuit sous forme d'un ou de plusieurs canaux auto-moulés dans la gaine de tuyau flexible, **en ce que** de préférence la section transversale totale minimale de tous les canaux moulés dans la gaine de tuyau flexible en tant que lumières secondaires est égale ou supérieure à la section transversale maximale de la lumière secondaire annulaire dans la zone proximale du ballonnet.

**9.** Dispositif selon l'une des revendications précédentes, **caractérisé en ce qu'**un canal collecteur annulaire se trouve dans la zone de l'extrémité de tube proximale avec lequel toutes les lumières secondaires ou tous les canaux moulés en tant que lumières secondaires dans la gaine de tuyau flexible communiquent.

**10.** Dispositif selon l'une des revendications précédentes, **caractérisé en ce qu'**un raccordement communicant avec toutes les lumières secondaires pour un tuyau de remplissage est prévu au niveau de l'extrémité de tube ou au niveau du canal collecteur annulaire.

**11.** Dispositif selon l'une des revendications précédentes, **caractérisé en ce que** le réservoir volumique extracorporel (9)

a) présente à l'état librement déployé un volume supérieur au ballonnet intracorporel (4) ou au ballonnet d'étanchéification en forme de manchette dans la zone distale du tube, et/ou
b) est soumis à une pression constante ou approximativement constante, par exemple par un poids ou un élément à ressort.

**12.** Dispositif selon l'une des revendications précédentes, **caractérisé par** un système servant à la commande active ou à la régulation de la pression dans le réservoir volumique extracorporel qui peut être réalisé de telle sorte que la pression dans le ballonnet intracorporel (2) ou dans le ballonnet d'étanchéification en forme de manchette est maintenue constante.

**13.** Dispositif selon la revendication 12, **caractérisé par** un système de mesure pour la pression dans le ballonnet intracorporel (2) ou dans le ballonnet d'étanchéification en forme de manchette à des fins de prédéfinition de valeur réelle pour un circuit de réglage qui agit sur la pression dans le réservoir volumique extracorporel.

**14.** Dispositif selon la revendication 12 ou 13, **caractérisé en ce que** le réservoir volumique extracorporel (9) est réalisé sous forme de réservoir de type soufflet ou piston (37) qui peut être actionné par un entraînement (38), soit pour déplacer du volume vers le ballonnet (2) ou en prélever du ballonnet (2).

**15.** Dispositif selon l'une des revendications précédentes, **caractérisé en ce que** le ballonnet (2)

a) est de forme annulaire fermée ou réalisé sous forme toroïdale, et/ou
b) est réalisé sous forme de masque laryngé, en particulier pour l'application sur l'hypopharynx.

**16.** Dispositif selon l'une des revendications précédentes, **caractérisé par** un profilé (41) qui ne peut pas s'affaisser à l'intérieur du ballonnet (2) ou tige qui, en cas d'une contraction péristaltique de l'organe creux, dérive une partie du fluide de remplissage des segments de ballonnet de manière distale par rapport à l'onde de contraction dans des zones de manière proximale par rapport à l'onde de contraction, en ce que le profilé qui ne peut pas s'affaisser communique avec le ballonnet (2) et/ou sa conduite d'alimentation et/ou lumière de remplissage.

**17.** Dispositif selon l'une des revendications précédentes, **caractérisé en ce que** le clapet antiretour à direction de flux (26) ou l'élément d'étranglement sans direction de flux (27) ou les deux est ou sont disposés de manière extracorporelle.

Fig.1

Fig.1a

Fig.2

6

4a

SB

2

4b

10

SR

Fig.2a

SP

4a

4b

10

SR

Fig.2b

11

10

4a

4b

Fig.2c

ID

OD

SR

G

Fig.2d

13

4a

IIe

10

IIe

4b

13

Fig.2e

10

4b

13

14

Fig.3

12

SP

2

Fig.3a

12

SP

2a

2b

## Fig.4

18

IVa

15  16

12

IVa

17

## Fig.4a

20

12

18

19

## Fig.5

21

23

22

6

15

13

2

10

Fig.6

7

26

27

3

2

Fig.7

28

30

29

31    32

Fig.7a

P

DP

V

IBV

Fig.7b

30

30

31

Fig.8

Fig.9

Fig.10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1442765 A1
- WO 2005120618 A1
- US 20130146062 A1
- US 4762129 A
- EP 1061984 B1
- EP 0929339 B1